# EUROPÄISCHE PATENTSCHRIFT

(11) **EP 1 075 844 B1**
(45) Veröffentlichungstag und Bekanntmachung des Hinweises auf die Patenterteilung: **04.10.2006**
(21) Anmeldenummer: 00117069.5
(22) Anmeldetag: 09.08.2000
(51) Int. Cl.: A61M 1/36

(54) **Vorrichtung zur autologenen Transfusion von Blut**
Device for autologous blood transfusion
Dispositif de transfusion autologue du sang

(30) Priorität: 12.08.1999 DE 19938287
(43) Veröffentlichungstag der Anmeldung: 14.02.2001
(73) Patentinhaber: Fresenius AG, 61352 Bad Homburg v.d.H. (DE)
(72) Erfinder: Biesel, Wolfgang, Dr., 66564 Ottweiler (DE)
(74) Vertreter: Luderschmidt, Schüler & Partner

(56) Entgegenhaltungen:
- WO-A-97/15399
- BE-A- 1 005 193
- DE-A- 4 226 974
- DE-C- 4 227 695
- US-A- 4 243 531
- US-A- 5 744 047
- US-A- 5 919 125

## Beschreibung

Die Erfindung betrifft eine Vorrichtung zur autologenen Transfusion von Blut, die über eine Zentrifugeneinheit zur Aufnahme eines Autotransfusionssets verfügt. Darüber hinaus betrifft die Erfindung ein Autotransfusionsset für eine derartige Vorrichtung, die eine Separationseinheit zur Konzentration einer Zellfraktion und ein Schlauchsystem umfaßt.

Es sind verschiedene Vorrichtungen und Verfahren zur Gewinnung von Konzentraten aus bestimmten Blutkomponenten bekannt. Zur Gewinnung eines Thrombozytenkonzentrats beispielsweise wird Blut eines Spenders im extrakorporalen Kreislauf einer Zentrifugation unterworfen und in seine Bestandteile getrennt. Eine Vorrichtung zur Durchführung eines derartigen Verfahrens ist beispielsweise aus der DE 42 27 695 A1 bekannt.

Thrombozytenkonzentrate werden zur Behandlung von thrombozytopenischen Patienten benötigt. Obwohl die Abtrennung von Leukozyten durch Zentrifugieren im allgemeinen ausreichend ist, werden die Leukozyten, die eine Immunantwort zu vermitteln in der Lage sind, bei Transfusionen von Fremdblut eliminiert, um eine Immunisierung des Patienten auf jeden Fall zu vermeiden.

Neben der Transfusion von Fremdblut hat die intraoperative Autotransfusion breite Anwendung gefunden, bei der das autologe Blut, also eigene Blut des Patienten während der Operation gesammmelt, aufbereitet und retransfundiert wird. Die Vorteile der Transfusion von autologenen Blut liegen in der Vermeidung von Infektionskrankheiten, wie beispielsweise Aids, Hepatitis sowie von Transfusionsreaktionen aufgrund biologischer Inkompatibilität und Immunsystemreaktionen. Anwendung im Bereich der intraoperativen Autotransfusion finden sogenannte Vollblutretransfusionsverfahren, die das gesammelte Blut lediglich einer Partikelfiltration unterziehen, bis hin zu Plasmaseparations-/Waschverfahren, die gewaschene Erythrozytenkonzentrate zur Reinfusion bereitstellen.

Eine autologene Bluttransfusionsvorrichtung ist beispielsweise aus der WO99/02269 bekannt.

Während bei der Transfusion von Fremdblut die Gefahr von Immunreaktionen besteht, sind bei der Autotransfusion Immunreaktionen ausgeschlossen. Andererseits können physiologische Reaktionen nicht ausgeschlossen werden, da die Leukozyten bei der Sammlung und Lagerung traumatisiert und/oder aktiviert werden. Darüber hinaus hat sich gezeigt, daß mittels Leukozytendepletionsfilter auch Tumorzellen eliminiert werden können.

Die DE 42 27 695 C1 beschreibt eine Zentrifuge zum Auftrennen von Blut in seine Bestandteile, bei der in einem Sammelschritt Erythrozyten und Plasma in einem Speichergefäß zwischengespeichert werden.

Aus der US 5,744,047 ist ein Leukozyten-Filter bekannt, der auch für die autologene Bluttransfusion Vewendung finden soll. Die US 5,744,047 beschreibt eine Anordnung mit einer von dem Patienten zu einer künstlichen Lunge führenden Blutzuführleitung und einer von der künstlichen Lunge zu dem Patienten führenden Blutabführleitung. Der Leukozytenfilter soll sowohl in der Blutzuführt- als auch -abführleitung angeordnet werden. In der Blutzuführleitung ist stromauf des Leukozytenfilters noch ein Blutsammelbehälter (Kardioreservoir) vorgesehen.

Die BE1005193 beschreibt ein Transfusionset, das über einen Blutsammelbeutel verfügt, zu dem eine Blutzuführleitung führt und von dem eine Rückführleitung abgeht. In die Blutzuführleitung ist ein weiterer Beutel mit einem Leukozytenfilter geschaltet. Der Blutsammelbeutel ist über abtrennbare Verbindungsstücke mit der Blutzuführ- und -rückführleitung verbunden. Zum Einlegen in eine Zentrifuge wird der Blutsammelbeutel von dem Schlauchset abgetrennt. Daher kann eine Zellfraktion nicht in einem kontinuierlichen Prozess konzentriert werden.

Der nächstliegende Stand der Technik, WO 97/15399, beschreibt eine Blutzentrifuge, die zur autologenen Transfusion von Blut geeignet ist, und in deren Zuleitung ein Maschenfilter geschaltet ist. Dieser Filter ist nicht zur Elimination von Leukozyten bestimmt.

Der Erfindung liegt die Aufgabe zugrunde, eine Vorrichtung zur autologenen Transfusion von Blut mit einer Zentrifugeneinheit zur Aufnahme eines Autotransfusionssets und ein Autotransfusionsset für eine derartige Vorrichtung zu schaffen, mit denen die Sicherheit der autologenen Transfusion weiter erhöht wird.

Die Lösung dieser Aufgabe erfolgt erfindungsgemäß mit den im Patentanspruch 1 bzw. 2 angegebenen Merkmalen.

Zur Vermeidung von physiologischen Reaktionen durch aktivierte oder traumatisierte Leukozyten oder Metastasierung durch Tumorzellen ist in das Autotransfusionsset ein Filter zur Elimination von Leukozyten und/oder Tumorzellen integriert, die Immunreaktionen bzw. Metastasierungen verursachen können. Die Elimination von Leukozyten und/oder Tumorzellen kann mit den bekannten Leukozytendepletionsfiltern erfolgen, die neben Leukozyten auch spezifisch Tumorzellen binden können. Darüber hinaus werden mit den Leukozytendepletionsfiltern auch partikuläre Verunreinigungen eliminiert.

Die Elimination von Leukozyten und/oder Tumorzellen kann prinzipiell vor oder nach der Aufbereitung des Blutes erfolgen. Die Elimination von Leukozyten vor der Aufbereitung reduziert jedoch den Gehalt an Produkten der Leukozytenaktivierung bzw.-traumatisierung des Blutproduktes zur Transfusion. Daher ist der Filter zur Elimination von Leukozyten und/oder Tumorzellen in der zu der Separationseinheit des Autotransfusionssets führenden Blutzuführleitung angeordnet.

Die bekannten Autotransfusionssets verfügen im allgemeinen über einen in der Blutzuführleitung angeordneten Sammelbehälter, der auch als Kardioreservoir bezeichnet wird. Der Filter zur Elimination von Leukozyten und/oder Tumor-; zellen ist in dem Sammelbehälter angeordnet. Dies ist insofern vorteilhaft, als ein separates Filtergehäuse nicht erforderlich ist. Mit der Anordnung des Leukozytendepletionsfilters in dem Sammelbehälter entfällt nicht nur die Notwendigkeit, ein separates Filtergehäuse vorzusehen, sondern es wird auch die Gefahr von Leckagen aufgrund zusätzlicher Verbindungsstellen vermieden. Der Filter zur Elimination von Leukozyten und/oder Tumorzellen kann in dem Sammelbehälter zusammen mit dem Filter angeordnet werden, der bei den bekannten Kardioreservoirs im allgemeinen zur Entfernung von partikulären Verunreinigungen bereits vorhanden ist. Insofern ist der Fertigungsaufwand gering.

Wenn das autologe Blut vor dem Sammeln in dem Behälter gefiltert wird, ist bei Bedarf eine schnelle Retransfusion möglich. Die Anordnung des Filters in der zum Patienten führenden Blutrückführleitung limitiert hingegen den Fluß der dem Patienten zu retransfundierenden Zellfraktion. Der Filter zur Elimination von Leukozyten und/oder Tumorzellen sollte eine möglichst große Filteroberfläche aufweisen, so daß in kurzer Zeit große Blutvolumina von Leukozyten und/oder Tumorzellen befreit werden können.

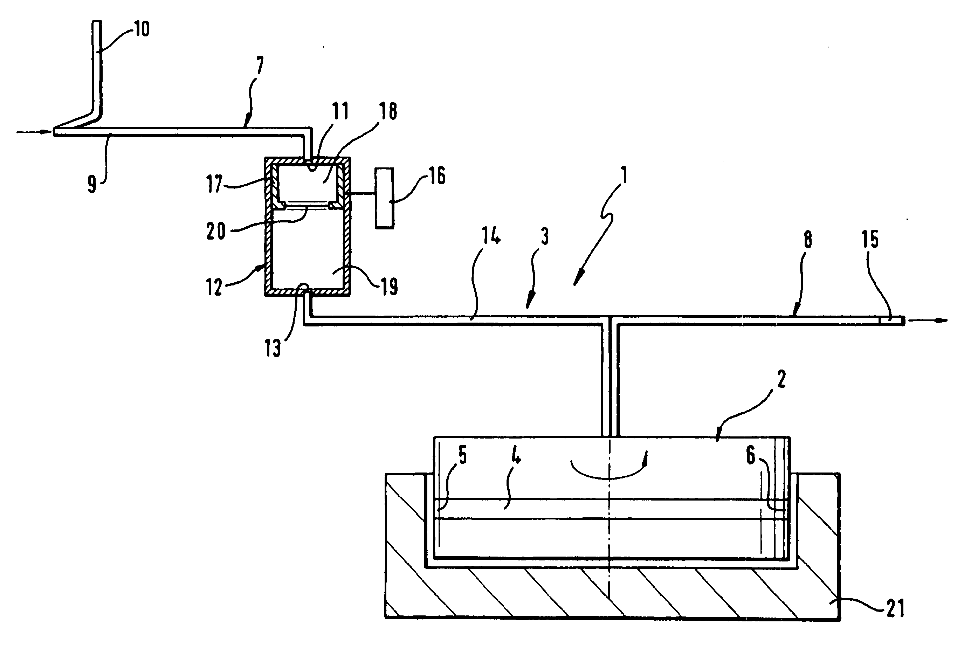

Im folgenden wird ein Ausführungsbeispiel einer Vorrichtung zur autologenen Transfusion von Blut zusammen mit dem Autotransfusionsset unter Bezugnahme auf die Zeichnung näher erläutert, die die Vorrichtung zur autologenen Transfusion zusammen mit dem Autotransfusionsset zeigt.

Die Vorrichtung zur autologen Transfusion und das Autotransfusionsset bilden zwei selbständige Einheiten. Zur Autotransfusion wird das als Disposable ausgebildete Autotransfusionsset in die Vorrichtung zur autologenen Transfusion eingelegt.

Die einzige Figur zeigt die wesentlichen Komponenten einer Vorrichtung zur autologenen Transfusion zusammen mit dem Autotransfusionsset in stark vereinfachter Darstellung. Das Autotransfusionsset 1 umfaßt eine Separationseinheit 2 zur Konzentration einer Zellfraktion sowie ein Schlauchsystem 3 zum Anschluß an den Patienten.

Bei der Separationseinheit 2 handelt es sich um eine Zentrifugenkammer mit einem ringförmigen Kanal 4, der einen Einlaß 5 für das aufzubereitende Blut und einen Auslaß 6 für die konzentrierte Zellfraktion, beispielsweise ein Erythrozytenkonzentrat, aufweist. Eine derartige Zentrifugenkammer ist beispielsweise in der DE 42 26 974 C2 im einzelnen beschrieben, auf die ausdrücklich Bezug genommen wird.

Das Schlauchsystem 3 des Autotransfusionssets umfaßt eine Blutzuführleitung 7 zum Zuführen des dem Patienten entnommenen Blutes und eine Blutrückführleitung 8 zur Retransfusion des in der Separationseinheit aufbereiteten Blutes.

Die Blutzuführleitung 7 führt zu dem Einlaß 11 eines Sammelbehälters 12 für das gesammelte, antikoagulierte Blut. Von dem Auslaß 13 des Sammelbehälters 12 führt ein zweiter Abschnitt 14 der Blutzuführleitung 7 zu dem Einlaß 5 des Separationskanals 4. Die Blutrückführleitung 8 ist an dem Auslaß 6 des Kanals 4 angeschlossen und weist an ihrem Ende einen Anschluß 15 zu einem nicht dargestellten Transferbeutel auf. Zum Ansaugen des Blutes ist der Sammelbehälter 12 mit einer nur andeutungsweise dargestellten Einrichtung 16 zur Erzeugung eines Unterdrucks verbunden. In den Sammelbehälter 12 ist ein Filtereinsatz 17 eingesetzt, der einen den Sammelbehälter in zwei Kammern 18,19 teilenden Filter 20 zur Elimination von Leukozyten und/oder Tumorzellen aufnimmt. Zum Zuführen eines Antikoagulanzmittels ist ein Anschluß 10 vorgesehen.

Die Autotransfusionsvorrichtung verfügt über eine Zentrifugeneinheit 21, in die die Separationseinheit 2 eingelegt ist. Das aufzubereitende Blut des Patienten strömt über den ersten Abschnitt 9 der Blutzuführleitung 7 in die erste Kammer 18 und über den Filter 20 in die zweite Kammer 19 des Sammelbehälters 12, wobei Leukozyten und/oder Tumorzellen zurückgehalten werden. Aus dem Sammelbehälter 12 strömt das von Leukozyten und/oder Tumorzellen befreite Blut dann über den zweiten Abschnitt 14 der Blutzuführleitung 7 in die rotierende Separationseinheit 2, in der eine Zellfraktion, beispielsweise die Erythrozytenfraktion, konzentriert wird. Das Erythrozytenkonzentrat wird dann über die Blutrückführleitung 8 zur Zwischenlagerung dem nicht dargestellten Transferbeutel zugeführt und wieder dem Patienten reinfundiert.

## Patentansprüche

1. Autotransfusionset für eine Vorrichtung zur autologenen Transfusion von Blut mit
einer in eine Zentrifugeneinheit einer Vorrichtung zur autologenen Transfusion von Blut einlegbaren Zentrifugenkammer (2) zur Konzentration einer Zellfraktion, die einen Einlass (5) für das aufzubereitende Blut und einen Auslass (6) für eine konzentrierte Zellfraktion aufweist, und einem Schlauchsystem (3), das eine mit dem Einlass (5) der Zentrifugenkammer (2) verbundene Blutzuführleitung (7) zum Zuführen des aufzubereitenden Bluts und eine mit dem Auslass (6) der Zentrifugenkammer verbundene Blutrückführleitung (8) für die konzentrierte Zellfraktion aufweist, wobei in der Blutzuführleitung ein Filter (20) angeordnet ist und die Blutzuführleitung (7) einen ersten Abschnitt (9) aufweist, der mit einem Einlass (11) eines Blutsammelbehälters (12) verbunden ist, und einen zweiten Abschnitt (14) aufweist, der mit einem Auslass des Blutsammelbehälters verbunden ist, und wobei der Filter (20) in dem Blutsammelbehälter (12) angeordnet ist, **dadurch gekennzeichnet dass** der Filter (20) zur Elimination von Leukozyten und/oder Tumorzellen geeignet ist, und der Blutsammelbehälter (12) mit einer Einrichtung (16) zur Erzeugung eines Unterdrucks derart verbunden ist, dass Blut in den Blutsammelbehälter (12) ansaugbar ist.

2. Vorrichtung zur autologenen Transfusion von Blut mit einer Zentrifugeneinheit (21), in die ein Autotransfusionsset nach Anspruch 1 eingelegt ist.

## Claims

1. An autotransfusion set for a device for the autologenous transfusion of blood with
a centrifuge chamber (2) for the concentration of a cell fraction, which centrifuge chamber is insertable into a centrifuge unit of a device for the autologenous transfusion of blood and has an inlet (5) for the blood to be prepared and an outlet (6) for a concentrated cell fraction, and a tube system (3) which comprises a blood supply line (7) for supplying the blood to be prepared, said blood supply line being connected to the inlet (5) of the centrifuge chamber, and a blood return line (8) for the concentrated cell fraction, said blood return line being connected to the outlet (6) of the centrifuge chamber, whereby a filter (20) is arranged in the blood supply line and the blood supply line (7) comprises a first segment (9), which is connected to an inlet (11) of a blood collecting container (12), and a second segment (14) which is connected to an outlet of the blood collecting container, and whereby the filter (20) is arranged in the blood collecting container (12), **characterised in that** the filter (20) is suitable for the elimination of leucocytes and/or tumour cells, and the blood collecting container (12) is connected to a device (16) for generating an underpressure in such a way that blood can be drawn by suction into the blood collecting chamber (12).

2. A device for the autologenous transfusion of blood with a centrifuge unit (21), into which an autotransfusion set according to claim 1 is inserted.

## Revendications

1. Kit d'autotransfusion pour un dispositif de transfusion autologue du sang avec une chambre de centrifugeuse (2) insérable dans une unité de centrifugeuse d'un dispositif de transfusion autologue du sang pour la concentration d'une fraction de cellule, qui présente une entrée (5) pour le sang à préparer et une sortie (6) pour une fraction de cellule concentrée, et un système de tuyaux (3) qui présente une conduite d'amenée du sang (7) reliée à l'entrée (5) de la chambre de centrifugeuse (2) pour amener le sang à préparer et une conduite de récupération du sang (8) reliée à la sortie (6) de la chambre de centrifugeuse pour la fraction de cellule concentrée, dans lequel un filtre (20) est placé dans la conduite d'amenée du sang (7)et celle-ci présente une première section (9) reliée à une entrée (11) d'un réservoir collecteur de sang (12) et présente une deuxième section (14) qui est reliée à une sortie du réservoir collecteur de sang et le filtre (20) est disposé dans le réservoir collecteur de sang (12), **caractérisé en ce que** le filtre (20) est approprié pour éliminer des leucocytes et/ou des cellules tumorales et le réservoir collecteur de sang (12) est relié à un dispositif (16) pour produire une sous-pression de sorte que du sang peut être aspiré dans le réservoir collecteur de sang (12).

2. Dispositif de transfusion autologue du sang avec une unité de centrifugeuse (21) dans laquelle un kit d'autotransfusion selon la revendication 1 est inséré.
